(19) Europäisches Patentamt
European Patent Office
Office européen des brevets

(11) Veröffentlichungsnummer: **0 287 954 B1**

(12) **EUROPÄISCHE PATENTSCHRIFT**

(45) Veröffentlichungstag der Patentschrift: **22.07.92**

(51) Int. Cl.5: **C07C 43/315**, C25B 3/02, C11B 9/00

(21) Anmeldenummer: **88105962.0**

(22) Anmeldetag: **14.04.88**

(54) **Neue Benzaldehyddialkylacetale, ihre Herstellung und Verwendung.**

(30) Priorität: **24.04.87 DE 3713732**

(43) Veröffentlichungstag der Anmeldung:
**26.10.88 Patentblatt 88/43**

(45) Bekanntmachung des Hinweises auf die Patenterteilung:
**22.07.92 Patentblatt 92/30**

(84) Benannte Vertragsstaaten:
**BE CH DE FR GB IT LI NL**

(56) Entgegenhaltungen:
**US-A- 2 450 878**
**US-A- 2 476 815**

(73) Patentinhaber: **BASF Aktiengesellschaft**
**Carl-Bosch-Strasse 38**
**W-6700 Ludwigshafen(DE)**

(72) Erfinder: **Degner, Dieter, Dr.**
**Kurpfalzstrasse 8**
**W-6701 Dannstadt-Schauernheim(DE)**
Erfinder: **Gramlich, Walter, Dr.**
**Auf der Hoehe 11**
**W-6803 Edingen-Neckarhausen(DE)**
Erfinder: **Lanzendoerfer, Franz, Dr.**
**Bruchstrasse 40**
**W-6701 Ellerstadt(DE)**
Erfinder: **Siegel, Hardo, Dr.**
**Hans-Purrmann-Allee 25**
**W-6720 Speyer(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die vorliegende Erfindung betrifft neue Benzaldehydderivate, ihre Herstellung und Verwendung als Riechstoffe und Riechstoffvorprodukte.

Acetale von Benzaldehyden finden aufgrund ihrer im Vergleich zu den Aldehyden erheblich größeren Stabilität in alkalischen Medien breite Anwendung in der Parfümerie, insbesondere bei der Parfümerierung von technischen Produkten. Bekannte Handelsprodukte sind u. a. Benzaldehyd-dimethylacetal (süßlich-grüner Duft), Benzaldehyd-diethylacetal (süßer, milder und grüner Duft), Anisaldehyd-dimethylacetal (herb-grüner, etwas blumiger Duft) sowie Anisaldehyd-diethylacetal (blumig-süßer, leicht grüner Duft).

Es wurde nun gefunden, daß die neuen Benzaldehyddialkylacetale der allgemeinen Formel

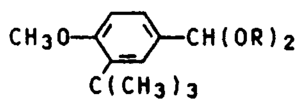

I,

in der R einen Alkylrest mit 1 bis 4 C-Atomen bedeutet, wertvolle Riechstoffe oder Riechstoffvorprodukte darstellen. R steht dabei z. B. für eine Methyl-, Ethyl, Propyl, iso-Propyl, Butyl- oder i-Butylgruppe.

So zeigt beispielsweise das Dimethylacetal des 3-tert.-Butyl-4-methoxi-benzaldehyds einen interessanten, blumig-süßen Duft mit einer erdigen Nebennote, das entsprechende Diethylacetal besitzt eine interessante langhaftende Grünnote.

Die neuen Acetale der Formel I lassen sich nach einer besonders eleganten Ausführungsform der Erfindung dadurch herstellen, daß man 3-tert.-Butyl-4-methoxi-toluol der Formel

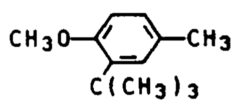

II,

in Gegenwart eines Alkanols der Formel ROH, in der R einen Alkylrest mit 1 bis 4 C-Atomen bedeutet, elektrochemisch oxidiert.

Als Alkanole verwendet man bevorzugt Methanol und Ethanol.

Das erfindungsgemäße Verfahren benötigt keine besondere Zellkonstruktion, es wird bevorzugt in einer ungeteilten Durchflußzelle durchgeführt. Als Anoden kommen z.B. Edelmetallelektroden, wie Pt oder Oxidelektroden, wie $Ti/RuO_2$ in Betracht. Bevorzugtes Anodenmaterial ist Graphit. Als Kathoden kommen z.B. Stahl, Eisen, Nickel, Kupfer, Zink und Kohle sowie Edeletalle wie Pt in Betracht. Bevorzugtes Kathodenmaterial ist Graphit. Der Elektrolyt setzt sich aus der Ausgangsverbindung der Formel II, dem Alkanol und einem Leitsalz zusammen. Als Leitsalze kommen Neutralsalze, Säuren und Basen in Betracht. Beispiele für Neutralsalze sind Fluoride, wie KF. Sulfonate, wie $NaSO_3C_6H_5$, Sulfate wie $(CH_3)_4NSO_4CH_3$, Tetrafluorborate, wie $NaBF_4$, Phosphate und Phosphonate. Beispiele für Säuren sind Schwefelsäure, Alkyl- und Arylsulfonsäuren. Als Basen dienen z. B. Alkoholate, wie $NaOCH_3$ oder Hydroxide der Alkalimetalle, wie KOH.

Der Elektrolyt hat beispielsweise folgende Zusammensetzung 3 bis 60 Gew.% Verbindung der Formel II
35 bis 90 Gew.% ROH
0,5 bis 10 Gew.% Leitsalz.

Die Stromdichte kann bei dem erfindungsgemäßen Verfahren in weiten Grenzen, z. B. von 0,5 bis 20 $A/dm^2$ gewählt werden. Bevorzugt wird bei 1 bis B $A/dm^2$ gearbeitet. Die Temperaturen bei der Elektrolyse betragen z.B. 20 bis 60 ° C. Die Elektrolyse wird bevorzugt bei Normaldruck durchgeführt. Die Ausgangsverbindung 11 läßt sich weitgehend umsetzen. Unumgesetztes 3-tert.-Butyl-4-methoxi-toluol kann bei Bedarf zur Elektrolyse zurückgeführt werden. Die Elektrolyse kann sowohl diskontinuierlich als auch kontinuierlich durchgeführt werden.

Die Aufarbeitung der Elektrolyseausträge erfolgt nach an sich üblichen Methoden. Reagiert der Elektrolyseaustrag sauer, so wird er zunächst mit Basen, wie $NaOCN_3$ neutralisiert. Überschüssiges Alkanol wird abdestilliert. Das Leitsalz wird abfiltriert und kann zusammen mit dem Alkanol zur Elektrolyse rückgeführt werden. Die Rohacetale können z.B. durch Rektifikation weiter gereinigt werden.

Die neuen Acetale lassen sich auf an sich bekanntem Wege durch Hydrolyse mit Wasser zum 3-tert.-Butyl-4-methoxi-benzaldehyd umsetzen. 3-tert.-Butyl-4-methoxi-benzaldehyd ist ein gesuchter Riechstoff (empyreumatische Duftnote vom "Russisch-Leder-Typ"), der bisher aus 3-tert.-Butyl-4-methoxi-toluol durch Braunsteinoxidation in schwefelsaurer Lösung hergestellt wurde (US 2450878, 2476815). Da bei diesen Verfahren erhebliche Mengen an Abfallsalzen anfallen, eröffnet die vorliegende

Erfindung durch die neuen Acetale, die sich auf einfache Weise mit Wasser in den 3-tert.-Butyl-4-methoxi-benzaldehyd überführen lassen, einen neuen, besonders vorteilhaften Weg zur Herstellung dieses Riechstoffes.

Beispiel 1

Elektrosynthese von 3-tert.-Butyl-4-methoxi-benzaldehyddimethylacetal

| | |
|---|---|
| Apparatur: | ungeteilte Zelle mit 11 Elektroden |
| Anoden: | Graphit Elektrolyt; 1729 g 3-tert. -Butyl-4-methoxi-toluol |
| | 60 g $KSO_3C_6H_5$ |
| | 10140 g $CH_3OH$ |
| Kathoden: | Graphit |
| Stromdichte: | 3.4 A/$dm^2$ |
| Elektrolysetemperatur: | 26 °C |

Elektrolyse mit 5,2 F/Mol 3-tert.-Butyl-4-methoxi-toluol
Der Elektrolyt wird während der Elektrolyse mit 200 1/h durch die Zelle über einen Wärmetauscher gepumpt.

Aufarbeitung:

Nach Beendigung der Elektrolyse wird Methanol bei Normaldruck bis zu Sumpftemperaturen von 120 °C abdestilliert. Der Rückstand wird bei 40 bis 50 °C über eine Drucknutsche filtriert. Hierbei erhalt man 75 g lösungsmittelfeuchtes Salz, das zusammen mit dem Methanol zur Elektrolyse zurückgeführt werden kann. Das Filtrat wird bei 6 mbar (Kopfdruck) und 80 bis 125 °C fraktioniert destilliert. Hierbei erhält man 98.7 g 3-tert.-Butyl-4-methoxi-toluol und 159,4 g 3-tert.-Butyl-1-methoximethyl-4-methoxibenzol, die beide zur Elektrolyse zurückgefuhrt werden können, sowie 1408,7 g 3-tert.-Butyl-4-methoxi-benzaldehyddimethylacetal.

| | |
|---|---|
| HNMR ($CDC1_3$): | 1.36 ppm (s) -$C(CH_3)_3$, |
| | 5.32 ppm (s) -CH = |
| | 3.33 ppm (s) = $C(OCH_3)_2$, |
| | 6.86 ppm (d) arom. H |
| | 3.85 ppm (s) $ArOCH_3$, |
| | 7,24 -7.33 ppm (m) arom. H |
| $n_D^{20}$ : | 1,4978 |

Hieraus errechnen sich ein Umsatz, bezogen auf 3-tert.-Butyl-4-methoxi-toluol. von 94.3 %, eine Ausbeute an 3-tert.-Butyl-4-methoxi-benzaldehyd-dimethylacetal von 60,9 % und eine Selektivität für 3-tert.-Butyl-4-methoxi-benzaldehyddimethylacetal von 70,5 %.

Beispiel 2

Elektrosynthese von 3-tert.-Butyl-4-methoxi-benzaldehyddiethylacetal

| | |
|---|---|
| Apparatur: | ungeteilte Zelle mit 11 Elektroden |
| Anoden: | Graphit |
| Elektrolyt: | 450 g 3-tert.-Butyl-4-methoxi-toluol |
| | 15 g $NaSO_3C_6H_5$ |
| | 2535 g $C_2H_5OH$ |
| Kathoden: | Graphit |
| Stromdichte: | 1.7 A/$dm^2$ |
| Elektrolysetemperatur: | 45 °C |

Elektrolyse mit 4.2 F/Mol 3-tert.-Butyl-4-methoxi-toluol
Der Elektrolyt wird mit 400 1/h während der Elektrolyse über einen Wärmetauscher durch die Zelle

gepumpt.

Aufarbeitung

Nach Beendigung der Elektrolyse wird Ethanol bei Normaldruck bis zu Sumpftemperaturen von 140° C abdestilliert, der Rückstand über eine Drucknutsche bei 30 bis 40° C filtriert (Rückstand, lösungsmittelfeucht: 21 g) und das Filtrat bei 4 mbar (Kopfdruck) und 119 bis 122° C fraktioniert destilliert. Hierbei erhält man 21,7 g 3-tert.-Butyl-4-methoxi-toluol und 10,7 g 3-tert.-Butyl-1-ethoximethyl-4-methoxibenzol, die beide zur Elektrolyse zurückgeführt werden können. sowie 491,5 g 3-tert.-Butyl-4-methoxi-benzaldehyddiethylacetal $n_D^{25}$ : 1,4883

HNMR ($CDC1_3$): 1.25 ppm (t) $-CH_3$, 3.84 ppm (s) Ar-$OCH_3$
1.36 ppm (s) $-C(CH_3)_3$, 5.44 ppm (s) -CH=
3.47 - 3.68 ppm (m)-$CH_2$-,
6,85 ppm (d) arom. H 7.25. - 7.36 ppm (m) arom. H

Hieraus errechnen sich ein Umsatz. bezogen auf 3-tert.-Butyl-4-methoxi-toluol von 95.2 %. eine Ausbeute an 3-tert.Butyl-4-methoxi-benzaldehyddiethylacetal von 73.1 % und eine Selektivität für 3-tert.-Butyl-4-methoxi-benzaldehyddiethylacetal von 78,4 %.

Beispiel 3

Synthese von 3-tert.-Butyl-4-methoxi-benzaldehyd

Man erhitzt 99.4 g 3-tert.-Butyl-4-methoxi-benzaldehyddimethylacetal in 300 g Wasser 3 Stunden unter Rückfluß zum Sieden. Danach wird angekühlt und der Rückstand abfiltriert und getrocknet. Hierbei erhalt man 77,5 g 3-tert.-Butyl-4-methoxi-benzaldehyd (Fp. 52 bis 53° C) dies entspricht einer Ausbeute von 96,7 %.

**Patentansprüche**

**1.** Benzaldehyddialkylacetale der Formel

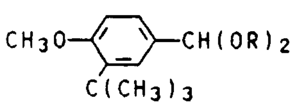

(I),

in der R eine Alkylgruppe mit 1 bis 4 C-Atomen bedeutet.

**2.** 3-tert.-Butyl-4-methoxi-benzaldehyddimethylacetal.

**3.** 3-tert.-Butyl-4-methoxi-benzaldehyddiethylacetal.

**4.** Verfahren zur Herstellung von Benzaldehyddialkylacetalen nach Anspruch 1, dadurch gekennzeichnet, daß man 3-tert.-Butyl-4-methoxi-toluolder Formel

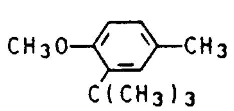

(II)

in Gegenwart eines Alkanols der Formel ROH, in der R eine Alkylgruppe mit 1 bis 4 C-Atomen bedeutet, elektrochemisch oxidiert.

**5.** Verfahren nach Anspruch 4, dadurch gekennzeichnet, daß man die Elektrooxidation in einer ungeteilten Zelle an Graphitelektroden durchführt.

6. Verfahren nach Anspruch 4, dadurch gekennzeichnet, daß man für die Elektrooxidation einen Elektrolyten heranzieht, der 3 bis 60 Gew.% der Verbindung der Formel II, 35 bis 90 Gew.% eines Alkanols der Formel ROH und 0,5 bis 10 Gew.% eines Leitsalzes enthält.

7. Verfahren nach Anspruch 4, dadurch gekennzeichnet, daß man die Elektrooxidation bei einer Stromdichte von 0,5 bis 20 $A/dm^2$ und einer Temperatur von 20 bis 60 ° C durchführt.

8. Verwendung der Benzaldehyddialkylacetale gemäß Anspruch 1 als Riechstoffe oder als Riechstoffvorprodukte.

9. Verwendung der Benzaldehyddialkylacetale gemäß Anspruch 1 für die Herstellung von 3-tert.-Butyl-4-methoxi-benzaldehyd.

**Claims**

1. A benzaldehyde dialkyl acetal of the formula

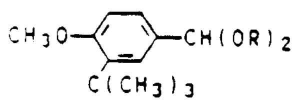

(I)

where R is alkyl of 1 to 4 carbon atoms.

2. 3-tert-Butyl-4-methoxybenzaldehyde dimethyl acetal.

3. 3-tert-Butyl-4-methoxybenzaldehyde diethyl acetal.

4. A process for preparing a benzaldehyde dialkyl acetal as claimed in claim 1, which comprises oxidizing 3-tert-butyl-4-methoxytoluene of the formula

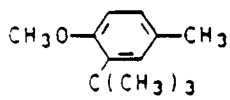

(II)

electrochemically in the presence of an alkanol of the formula ROH where R is alkyl of 1 to 4 carbon atoms.

5. A process as claimed in claim 4, wherein the electrooxidation is carried out in an undivided cell on graphite electrodes.

6. A process as claimed in claim 4, wherein the electrooxidation is performed using an electrolyte containing from 3 to 60% by weight of the compound of the formula II, from 35 to 90% by weight of an alkanol of the formula ROH and from 0.5 to 10% by weight of a conducting salt.

7. A process as claimed in claim 4, wherein the electrooxidation is carried out with a current density of from 0.5 to 20 $A/dm^2$ at from 20 to 60 ° C.

8. Use of a benzaldehyde dialkyl acetal as claimed in claim 1 as a scent or scent intermediate.

9. Use of a benzaldehyde dialkyl acetal as claimed in claim 1 for preparing 3-tert-butyl-4-methoxybenzaldehyde.

**Revendications**

**1.** Dialkylacétals de benzaldehyde de formule

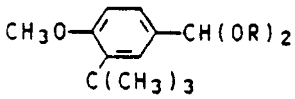

(I),

dans laquelle R représente un groupement alkyle à 1-4 atomes de carbone.

**2.** Diméthylacétal de 3-tert.-butyl-4-méthoxybenzaldéhyde.

**3.** Diéthylacétal de 3-tert.-butyl-4-méthoxybenzaldéhyde.

**4.** Procédé de préparation de dialkylacétals de benzaldéhyde selon la revendication 1, caractérisé en ce qu'an oxyde électrochimiquement du 3-tert.-butyl-4-méthoxytoluéne de formule

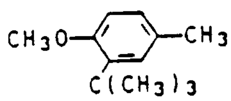

(II)

en présence d'un alcanol de formule ROH, dans laquelle R représente un groupement alkyle à 1-4 atomes de carbone.

**5.** Procédé selon la revendication 4, caractérisé en ce qu'on effectue l'electro-oxydation dans une cellule non divisée, sur des électrodes de graphite.

**6.** Procédé selon la revendication 4, caractérisé en ce qu'on utilise, pour l'électro-oxydation, un électrolyte qui contient de 3 à 60% en poids du composé de formule II, de 35 à 90% en poids d'un alcanol de formule ROH et de 0,5 à 10% en poids d'un sel conducteur.

**7.** Procédé selon la revendication 4, caractérisé en ce qu'on effectue l'électro-oxydation avec une densité de courant de 0,5 à 20 $A/dm^2$ et à une température de 20 à 60 ° C.

**8.** Utilisation des dialkylacétals de benzaldéhyde selon la revendication 1 comme parfums ou produits intermédiaires de parfums.

**9.** Utilisation des dialkylacétals de benzaldéhyde selon la revendication 1 pour la préparation de 3-tert.-butyl-4-méthoxybenzaldéhyde.